# EUROPEAN PATENT APPLICATION

(11) **EP 3 660 855 A1**
(43) Date of publication of application: **03.06.2020**
(21) Application number: 19212019.4
(22) Date of filing: 28.11.2019
(51) Int. Cl.: G16H 10/60, G06Q 50/00

(54) **MULTI-CHANNEL DATA AGGREGATION SYSTEM AND METHOD FOR COMMUNICATING ANIMAL BREED, MEDICAL, AND PROFILE INFORMATION AMONG REMOTE USER NETWORKS**

(30) Priority: 30.11.2018 US 201862773437 P; 30.11.2018 US 201862773441 P; 30.11.2018 US 201862773442 P; 06.12.2018 US 201862773444 P
(71) Applicant: TailTrax, LLC, Brentwood, TN (US)
(72) Inventor: Gelfand, Matt, Brentwood, Tennessee (US)
(74) Representative: De Bortoli, Eros

(57) **Abstract**

A multi-channel data aggregation system communicates non-human animal data among multiple remote user networks, wherein users are provided for non-human animals among user networks for each of caregivers, owners, and breeders. Unique user interfaces are respectively associated with each user network, and configured to link a hosted server network to respective user devices. The hosted server network associates primary stored data for an individual non-human animal with each respective caregiver, owner, and breeder, and further associates derivative stored data with at least familial non-human animal relations and their respective caregivers, owners and breeders. The server network also provides unique identifiers for each respective caregiver, owner, and breeder, enables users to provide selective access to corresponding primary and/or derivative stored data by other users, and, responsive to a user search request, produces accessible primary and/or derivative data corresponding to parameters in the search request.

## Description

The present invention relates generally to systems and methods for managing animal breed information and trend data from aggregate data points pertaining to one or more animals and breeding lineage thereof. More specifically, the present invention pertains to a computer-enabled distributed ledger and data management system for animal sources, owners, and veterinarians, and further facilitating interactions at least with respect to associated animals.

Analytics of medical information based upon genetic lineage and trend data have traditionally been limited in the medical world due to compartmentalization of breed information amongst non-human animal sources, owners, and veterinarians, separately. The term "source" in the context of animals as used herein may also refer to rescue entities or similar channels, but most commonly refers to animal breeders, and this term may be used generally hereinafter for simplicity.

Breeders, owners, and veterinarians lack an easy way to share pertinent information freely between other breeders, owners, and veterinarians. Further, traditional systems of sharing information are prone to programmatic or transcription errors. Additionally, custodians of animal medical information may be legally prohibited from sharing certain information due to medical or privacy laws, which limits the ability to analyze genetic and medical data pertaining to animals, breeds, and lineages.

Traditionally, breeders must rely on owners to contact them and provide them with information pertaining to an animal's medical diagnoses, which in turn must be communicated to the owner from a veterinarian. The separation and siloing of information limits the ability of breeders, owners, and veterinarians to make well-informed decisions regarding breeding, ownership, and medical care of animals, as such individuals are unlikely to have a comprehensive picture of breed, lineage, and animal history. A centralized repository of animal medical information provides some benefit to breeders, owners, and veterinarians by encouraging the sharing of pertinent information. However, centralized repositories are limited in their benefits and require ongoing management and accurate input for all three user-types in order to maintain data veracity. Any one failure to report data properly can call into question whether certain transactions or analyses occurred. For example, an owner may report purchasing an animal from a breeder, but a breeder fails to report the sale of the animal to the owner, thus calling into question whether the sale of the animal was authorized.

Also, while present technologies exist for automatically detecting and classifying human athletic activities via hardware sensory inputs, such technologies are limited in application to animal-related activities. Activity trackers do not determine the difference between a user briskly walking and a user walking his or her dog. Nor do activity trackers take into consideration pet-related behaviors such as whether the animal stops frequently or tugs hard on the leash. Such information would be useful to collect and aggregate to provide feedback to users regarding pet performance, such as pet leash behavior, speed, fitness, activity, mood, and the like.

It would be desirable to facilitate aggregation of pet-related activity and behavior, which may further provide non-owner users beneficial information regarding animal health and activity information. For example, data mining of the behavioral and activity information may demonstrate heretofore unknown information about pet behaviors and activities that may improve the quality of pet-related products and services. Detecting pulling behaviors in large-breed dogs may suggest certain types of harnesses are more or less beneficial at training said large-breed dogs on proper leash behavior. Monitoring animal activity over time may reveal correlations between animal endurance and animal longevity. Animals who eat too quickly may be correlated with a percentage of higher likelihood of stomach flipping or esophageal cancer. Aggregating traditionally disparate or non-collected pet information may desirably enable users to determine beneficial activities and change habits or decisions, providing favorable results with pets and animal care.

Another issue where limited availability of animal ownership information creates costly problems is in reuniting lost-and-found pets with their owners. Finders of runaway or lost pets may traditionally post on social media channels or other information boards the identity of a found pet, but, unfortunately, such good Samaritans often fall victim to fraudulent claims of ownership. For example, some individuals lie and claim that they are the true owners of the pets in order to steal the pet, either for personal reasons or for use in illegal industries such as dogfighting. Because the pet finders have no reliable means for verifying pet owner identities, many must surrender the animal to the claimed owner without any means of verification beyond the alleged owner's word. While subcutaneous information chips have attempted to address this issue, such chips are often incorrect, lack proper contact information, and can even compound the problem by introducing uncertainty if the chip information is incorrect or incomplete and does not match the proper owner attempting to reclaim the pet.

Accordingly, it may further be desirable to facilitate the sharing, aggregation, and analysis of animal ownership and lineage information from an animal's three primary care providers-breeders, owners, and veterinarians- and encourage the sharing of information through the formation and use of social media and online marketplace platforms.

The present invention intends to respond to the above-mentioned needs by providing a multi-channel data aggregation system according to the following claim 1 and related dependent claims.

In an embodiment as disclosed herein, a multi-channel data aggregation system is provided for communicating non-human animal data among a plurality of remote user networks, wherein at least one user is provided for a particular non-human animal among each of a caregiver user network, an owner user network and a breeder user network. A plurality of user interfaces are respectively associated with each of the caregiver user network, the owner user network and the breeder user network, wherein each user interface links a hosted server network to one or more user devices for the respective user network, and enables user entry of primary data for individual non-human animals, said primary data stored in accessible association with the hosted server network. The hosted server network associates primary stored data for an individual non-human animal with each respective caregiver, owner, and breeder, and further associates derivative stored data with at least familial non-human animal relations and their respective caregivers, owners and breeders. The hosted server network also provides unique identifiers for each respective caregiver, owner, and breeder, and enables any one or more users to provide selective access to corresponding primary and/or derivative stored data by a specified one or more of the other users. Responsive to a user search request from any one or more of the user interfaces, the hosted server network further produces accessible primary and/or derivative data corresponding to parameters in the search request.

In a particular exemplary embodiment, the hosted server network comprises a decentralized network platform, and is further configured to generate a distributed ledger stored across a plurality of nodes, and to provide unique identifiers in the distributed ledger for each respective caregiver, owner, and breeder.

In one aspect, upon confirmation of a transaction between a plurality of users, the decentralized platform uploads and verifies at least sale and brood information via corresponding ledger nodes, wherein at least the brood information comprises primary and/or derivative data associated with animals in the transacted brood.

Such distributed ledger systems may implement blockchain technologies to facilitate the storage, management, transfer, and verification of animal information such as breed, health, medical associated care provider, and other such information to be shared between a plurality of animal care providers. For example, the distributed ledger systems and methods may enable computer-assisted transactional activities with respect to contracting for the sale or stud rights of an animal; performing and purchasing veterinary care services; analyzing various animal data sets while maintaining end-user and animal privacy; enabling review of animal-related services and quality, and the like. Such transactions may benefit prospective owners by providing animal, lineage, and breed information verified via multiple distributed sources; may benefit owners by enabling crowdfunding or other distributed methods of financing animal care procedures, and may benefit veterinarians by providing auditable medical records for animals, among other benefits.

In another aspect, the decentralized platform may further programmatically identify animal and user profiles associated with the transaction, and update the sale and brood information accordingly. An exemplary decentralized network media platform may aggregate and record from animal care providers ("users") information pertaining to animals, including animal lineage, genealogy, relatedness, and medical history. The aggregated information may be stored on a disassociated ledger stored upon multiple user and/or non-user computers. In various embodiments, user information may further be stored in associated with the distributed ledger. User information may include an encrypted blockchain "wallet" for secure identification and authorization of the user upon the decentralized network media platform. The decentralized system may enable verifiable programmatic transactions involving user and animal data, including the sale of animals and animal-related services and the analytical use of user and animal data. The system may further enable the exchange of data and messaging, the recording thereof cryptographically protected but stored upon the distributed ledger. Such transactions are programmatically enabled via algorithmically managed inputs to be verified via the decentralized network media platform's nodes that store the disassociated ledger.

User information may include one or more user profiles in association with one or more animal profiles. User profiles may include data such as name, login, public and private keys, address, contact information, user type, associated animal profiles, financial balance, services provided, etc. Animal profiles may include animal medical and non-medical data, including, for example, name, type, breed, birthdate, current and prior associated user information, familial relationship data, medical records, pet microchip number, photos, location history, personality characteristics, disposition, dietary information, breed traits, etc. Animal profiles may further be genealogically associated with other animal profiles on basis of similar litter, parentage, and animal ownership.

The decentralized network media platform may perform desirable transactional facilitation interaction between users and record such transactions into the disassociated ledger stored across multiple nodes. For example, the system may enable users to contract for the sale of an animal's brood upon production, whereby the sale and brood information are uploaded to and verified by the ledger nodes. In such examples, the system may further programmatically identify related animal and user profiles and update the data accordingly. For example, the production of a litter may automatically verify and populate the data of associated relative animal profiles with the new litter information; the subsequent diagnosis of one pup of the litter with a disease may update the associated animal profiles with such information, such as, for specific example, to identify the relatively likelihood that a lineage is prone to a genetic condition.

In another aspect, the platform may be configured to require verification of one or more digital certificates in association with the transaction, wherein the confirmed transaction comprises a cryptocurrency transaction such as bitcoin or other coin-related cryptographical valuation transfer methodologies. Such cryptocurrency transactions may enable various users to contract for goods and services within the platform. Financial and/or non-financial transactions may be grouped into blocks to be stored upon the blockchain following node verification. Such blockchain node verification may occur in accordance with antifraud and other data verification techniques to prevent transactional discrepancies or node hijacking. For example, the platform may enable for a decentralized server but otherwise require verification of short-term digital certificates, whereby nodes and users must verify access to the platform periodically in order to continue to use, access, and store the blockchain data.

In another aspect, the platform may be configured, responsive to the user search request from any one or more of the user interfaces, to decrypt selectively accessible primary and/or derivative data via a master key and provide an output report to the requesting user without providing the decrypted data. For example, sensitive data such as personal health information or personally identifiable information, such as a user's name and contact information or a pet's health information, may be cryptographically hashed and obfuscated. In one embodiment, an associated user may selectively enable what kind and how much data to reveal to other users, either specifically or in aggregate. Such selective sharing of information may occur via one or more public/private key exchanges for decryption of the stored data to be exchanged or shared. In one embodiment, a master decryption key may be stored in accordance with the platform software, thereby enabling an authorized software administrator to access and view all user and animal profile data. In an alternative embodiment, keys may be fully decentralized and managed by each user.

Keys may be uniquely identified by or associated with an individual animal. For example, an animal may be identified and managed in association with a unique number. In an embodiment, the unique animal number may be derived from one or more biometric identifiers whereby one or more identifier-generation algorithms generates a unique key on the basis of a biometric input. Namely, the platform may be able to detect a unique nose print for an animal from a data input such as a nose scan, a high-resolution picture, a nose/fingerprint reader, etc. In an embodiment, the platform may be able to detect a unique iris pattern using the same or similar input vectors. In a further embodiment, the platform may associate the unique animal number with the public or private decryption key of the user. In a further embodiment, another element of the public and/or private key may be user-specific, such as a key or number generated from a user-biometric input. Accordingly, the animal biometric input may be required as part of the cryptographic encryption/decryption process for the platform.

The decentralized platform may provide for anonymous, pseudonymous, and/or verified messaging between one or more users. Such messaging may include direct messages, such as medical alerts generated by veterinarians to owners and, potentially, associated breeders. Medical alerts may be distributed in association with animal and user relationships; for example, a medical alert generated by one veterinarian or breeder in association with an animal may be sent real-time to one or more users associated with animals itself associated with the alert-related animal, such as owners of littermates receiving an alert that one puppy from the litter was diagnosed with Parvo. Messaging may further include reviewing and rating of users and animals whereby the platform algorithmically determines from the user review data comparatively good and bad users, animals, and animal lineages. The review information may be user-accessible to enable users to make informed transactional decisions, such as whether to breed two animals, purchase a pet from a specific breeder, or purchase veterinary services from a specific veterinarian.

The decentralized platform may enable the secure storage of animal health information including medical records. Distributed ledgering of such animal health information may enable users to share animal health information with various other users as needed, such as exchanging records between veterinarians, between owners and breeders, and the like.

Users may be able to search, aggregate, and analyze data stored upon the blockchain. In one embodiment, the platform may enable a user only to access data to which the user is cryptographically verified (i.e. signed keys). In another embodiment, the user may request one or more reports to be generated by the platform, which decrypts the data via a master key and provides the output report without providing the decrypted data to the requesting user. In still another embodiment, the system may permit users to search blockchain data in its encrypted state for certain trend information or other characteristics via homomorphic encryption. For example, a user owner may build a query that asks whether any relatives of an owned animal has been diagnosed with Cushing's Syndrome; the platform aggregates and analyzes the relative animal profile information to determine whether any medical records indicate a Cushing's Syndrome diagnosis is "TRUE," and reports back on the status of the result query.

Encryption protocols may vary in complexity and strength based upon the sensitivity of information protected. For example, sensitive information may be protected with stronger cryptographic algorithms that enable higher levels of processing power to decrypt or search, whereas non-sensitive information may use lightweight cryptographic protocols to decrease size and computational processing requirements. In another embodiment, certain data may not require cryptographic encryption at all. In still another embodiment, certain sensitive information may be protected within a threshold cryptosystem. For example, release of certain animal health information may require the release signatures of at least the owner and veterinarian. The platform may programmatically enable users to request for the release of information by the verified threshold users without directly identifying the users.

In another embodiment for providing animal activity-based feedback, a variant of the aforementioned multi-channel data aggregation system is configured to receive activity data associated with the given non-human animal via the at least one user interface, wherein the primary data comprises data manually entered via the at least one user interface and data automatically generated or derived from one or more sensors positioned in association with the given non-human animal. The server detects a behavior or the likelihood of a behavior associated with the non-human animal, and provides one or more offers to the user via the user interface, based on the detected behavior or likelihood of the behavior.

In one aspect, the one or more offers may comprise one or more of: an interactive user experience corresponding to a type of detected behavior or likely behavior; a digital token associated with performance of the detected behavior or likely behavior; and a modified user experience element on the user device. For example, the platform may provide users with interactive user experiences dynamically based upon animal data input and determinations made therefrom. A user may be able to play an augmented reality game only when the system determines that an animal is being walked. This data may be shared collectively in part or in full such as to provide a social experience.

In another aspect, the hosted server network may be configured to associate detected behavior or likelihood of behavior with one or more animal-related products, and determine correlations between a type of detected behavior or likelihood of behavior and the one or more animal-related products. For example, the platform may identify key geolocational points where animals are determined to be likely to urinate and mark territory; the platform may within the context of a game identify this point as a "territory" to be "captured" by the pet and reward the user for walking to and tarrying at the point. In said gamified example, the user may further be rewarded with digital or physical assets such as badges or pet toys for the accumulation of points or proper performance. Said point accumulation and performance may, in an embodiment, be determined in association with positive and healthy activities such that the user and the pet are rewarded for beneficial actions taken. The sensory determinations and rewards may create a reward-based feedback loop for encouraging positive behaviors.

In another aspect, multiple user interfaces are respectively associated with each of a caregiver user network, an owner user network and a breeder user network, wherein each user interface is configured to link the host server to one or more user devices for the respective user network. A data storage network is functionally linked to the hosted server network and configured to associate primary stored data for individual non-human animals with each respective caregiver, owner and breeder, and to further associated derivative stored data with at least familial non-human animal relations and their respective caregivers, owners and breeders. The hosted server network is further configured to aggregate primary and derivative stored data with respect to each individual caregiver, owner and breeder and across each of one or more metrics specific to their respective user network, to determine a relative importance of the detected behavior or likelihood of behavior associated with the non-human animal for any one or more of the caregivers, owners, and breeders, and to deliver one or more messages comprising customized information regarding the detected behavior or likelihood of behavior to one or more of the caregivers, owners, and breeders, based at least in part on the determined relative importance for each potential recipient.

Such an embodiment generally enables various users to upload animal-related information via direct input and sensory input from one or more associated hardware devices and selectively share such information with users or user groups. The platform may further be configured to predict behavioral or other activity data and may use a neural network engine and machine learning algorithms to determine whether certain activities or behaviors are occurring based upon the data input. For example, the platform may predict when a user is walking a large dog, a small dog, no dog, a cat, or another animal. Said methodologies and systems may further predict when an animal has escaped from home, is pending escape from home, or is preparing to escape from home. The platform may make health-related determinations and make or otherwise assist users in making diagnoses.

Collected and aggregated data, and determinations therefrom, may be shared directly and in full, or data may selectively be shared such that other users may only have access to a subset or obfuscation of data. Further in said example, the user may allow limited access to certain animal information, such as allowing owners of associated animal relatives to know the type and severity of a medical diagnosis but not the diagnosis itself, or to allow unassociated users access to anonymized statistical data regarding the pet activity information in aggregate, such as seeing general distance walked but not timestamp or geostamp data.

In one aspect, the platform may be provided in the context of a social media platform, whereby users may perform e-commerce and ownership management activities in association with one or more animal profiles. For example, an animal profile for a discrete animal may be associated with one or more owner profiles, and the owner profile association may be transferred or modified in accordance with various transactional activities. Accordingly, breeder owners may be able to sell an animal online and transfer the ownership profile to the purchasing user. Ownership of an animal may be split between multiple users, such as spouses or co-habitants, or a large plurality of micro-owners who may own a fractional share of the animal, animal profile, and/or various rights in association with the animal such as breeding and stud rights.

In another aspect, the platform may further enable users or non-user researchers to access or review animal-related data based upon derived analyses of user and animal trend data. For example, the platform may be able to user image recognition, third-party APIs, hardware sensors, and the like to determine a relative rate, frequency, and distance that a dog is walked, compare such information to the dog's medical information, then determine trend data in aggregate to identify correlations and statistical information between rate, frequency, and distance of walking and a medical condition. For example, the system may determine that there is a high correlation between recovery from a femoral head ostectomy performed on German Shepherds under the age of three and long, slow, hill-climbing activities, but not as a high a correlation between such recovery and general walking distance. Such information-gathering and analysis may benefit users by enabling new data intelligence initiatives via pervasive and aggregate animal data collection, including information not traditionally collected in animal healthcare. Further, such data analysis may be particularly useful in the context of animal sports such as horseracing and greyhound racing, whereby data pertaining to animal performance in races may be analyzed in accordance with financial investments and predictions for blood-relatives, breeders, and trainers. Users may be able to glean new information from algorithmic analysis of corollary trends between high-performing animals and user- and animal-profile data.

In another aspect, a digital marketplace platform aggregates and records from animal care providers ("users") information pertaining to animals, including animal lineage, genealogy, relatedness, medical history, ownership, and ownership rights. This information may be aggregated from various sources such as electronic medical records, third-party systems, other social media platforms, hardware sensors, and the like. The platform may further collect information from users directly in the form of posts, messaging, or other types of affirmative submissions, such as in the context of a social media platform. For example, users may record walking times, moods, activities, pictures, thoughts, opinions, disposition, bowel movements, behavioral issues, etc. Users may further record, or the platform may import automatically, race results, ledgers, breeding data, stud books, training data, performance information, etc. The platform may combine this posted social media information with the other collected and aggregated data, such as sensory data, to form profiles for one or more users and one or more associated animals.

Animal profiles may be defined in accordance with information relating to a single animal, and such animal profiles may be associated with one another and defined based upon profile relationship. For example, two animals may be defined as biological relatives, litter-mates, cohabitants, siblings, enemies, etc. Animal profiles may further be associated with user profiles, which in turn may be associated with one another via relationship grouping. For example, an animal may be associated with one or more owners, custodians, veterinarians, veterinarian technicians, breeders, adoption agencies, fosters, trainers, walkers, groomers, care providers, and the like. Users may be further associated with one another in accordance with the animal-associated relationship, such as an owner who has contracted with a breeder and a custodian. In an embodiment, users may be associated with one another irrespective of an animal profile relationship, such as users who define themselves as siblings, relatives, friends, coworkers, etc. User profiles may include data such as name, login, public and private keys, address, contact information, user type, associated animal profiles, financial balance, services provided, etc. Animal profiles may include animal medical and non-medical data, including, for example, name, type, breed, birthdate, current and prior associated user information, familial relationship data, medical records, pet microchip number, photos, location history, personality characteristics, disposition, dietary information, breed traits, etc. Animal profiles may further include ownership, financial, and performance data. Animal profiles may further be genealogically associated with other animal profiles on basis of similar litter, parentage, and animal ownership.

In another aspect, the platform may provide for anonymous, pseudonymous, and/or verified messaging between one or more users. Messaging may be subject to one or more security, priority, or urgency levels. For example, health and financial information may be automatically encrypted, or, alternatively, a warning is provided to a user when sending such information over an unencrypted messaging channel. As another example, an owner may be able to send a high-priority message to a veterinarian user related to an animal's exhibition of highly concerning symptoms, such as vomiting and lethargy. In said example, the platform may treat high priority messages differently, such as to forward the message to the veterinarian's phone or to generate an audiovisual alert for the veterinarian or to override certain default messaging notification settings. The platform may further enable the secure storage of animal health information including medical records. Health-related messaging may be stored in association with the animal's medical record and animal profile.

In another embodiment, a further variant on the aforementioned multi-channel data aggregation system may be implemented to store and manage animal-related information and animal care provider information in association with a social media platform, including group structures defined therein. A data storage is functionally linked to the host server and configured to associate primary stored data for an individual non-human animal with respective caregivers, owners and/or breeders, and to further associate derivative stored data with at least familial non-human animal relations and their respective caregivers, owners and/or breeders. The social media platform is further configured to enable tagging by users via a respective user interface of selected primary and/or derivative data with respect to an associated animal, wherein the tags designate access by other individual users to the selected primary and/or derivative data. The social media platform further, responsive to a user request from any one or more of the user interfaces, produces primary and/or derivative data corresponding to the request and accessible to the requesting user.

The social media platform may be configured to provide a closed channel for animal-related messaging and contracting of transactions among at least first and second users via respective user interfaces, and/or an open channel for animal-related messaging and searchable posts for all users via respective user interfaces.

In one aspect, the tags may be configured to designate access by other individual users to one or more of: all of a selected first set of primary and/or derivative data, a subset of a selected second set of primary and/or derivative data, and an obfuscation of a selected third set of primary and/or derivative data. Primary and/or derivative data associated with a particular animal in this context may comprise medical information, a subset of said medical information comprising a set of primary and/or derivative data accessible by a first selected group of one or more users or a user network, and an obfuscation of said medical information comprising a set of primary and/or derivative data accessible by a second selected group of one or more users or a user network.

The social media platform may accordingly enable various users to upload animal-related information (e.g., activity data) from a local data source and selectively designate access to the uploaded animal activity data by a selected group of one or more users or a user network. Data may be shared directly and in full, or data may selectively be shared such that other users may only have access to a subset or obfuscation of data. For example, a pet owner may choose to enable selected sets of users to have access to a pet photograph (e.g. user relatives, user friends, associated breeder, associated veterinarian hospital users, owners of associated animal relatives) but only choose subsets of said users to have access to an animal's medical diagnosis (e.g. associated breeder, associated veterinarian hospital users).

Further in said example, the user may allow limited access to certain animal information, such as allowing owners of associated animal relatives to know the type and severity of a medical diagnosis but not the diagnosis itself, or to allow unassociated breeders access to anonymized statistical data regarding the animal breed/type and related diagnoses in aggregate. The platform may enable gamification or other psychological reward structures to promote the continuous uploading of animal-related and user-animal-related data.

The local data source may comprise one or more movement sensors configured to produce signals corresponding to movement characteristics of a given animal.

In one aspect, one or more owner user interfaces may be configured to display and transmit image data captured via an image capturing device of an associated owner user device, wherein the social media platform is selectively configured in association with owner user authorization to enable access by other users to the image data. Access to image data may be individually provided with respect to a given non-human animal or collectively provided with respect to the given non-human animal and familial relations thereto. The social media platform may further be configured to selectively display image data from the owner user interface on a caregiver user interface, based at least on a remote diagnostic request therefrom.

The system may enable selective enabling and disabling of access to animal data by authorized users. Accordingly, a first user may have the ability to invite a second user to a group that is authorized to access certain information, and then further "uninvite" them such that they can no longer access that same information. As one example, a dog owner takes his/her dog to an emergency veterinarian and invited them to a "family" or equivalent group having controlled access to the dog's health records. As another example, the dog owner may bring the dog to a boarder and invite the boarder to the group for as long as the pet was there. These actions in accordance with the disclosed system would enable the veterinarian/ boarder to see the dog's records in case of any medical emergencies. In each case, the access could be temporary in that the dog owner could selectively "uninvite" them when the dog was subsequently picked up.

In another aspect, the social media platform may determine current health conditions for a given animal based at least in part on analysis of the uploaded activity data and stored historical activity data and medical history. This information may be aggregated from various sources such as electronic medical records, third-party systems, other social media platforms, hardware sensors, and the like. For example, the social media platform may be configured to identify current health conditions for a given non-human animal as being independent to the given non-human animal, common to a breed associated with the given non-human animal, or potentially collective to familial relations of the given non-human animal. For identified current health conditions as potentially being collective with respect to familial relations, the social media platform may further generate notifications comprising accessible subsets or obfuscations of related primary and/or derivative data to user interfaces associated with familial relations of the non-human animal.

In one aspect, the social network media platform may facilitate transactional activities for one or more users, including between users. For example, the platform may enable users to upload text, images, and other information pertaining to an animal; to share said information with others; to contract with other users with respect to animal-related transactions (e.g. animal care, pet-sitting, grooming, adoption, etc.). Such information may be protected from disclosure such that one or more users must provide permission before sharing animal or user information. For example, a user may affirmatively post information and choose which users and/or groups with which to share the information, may non-affirmatively share said information based upon the user's privacy settings, or may share said information upon the approval of one or more other users, such as a veterinarian or breeder.

The platform may further provide for user-to-user messaging, including audiovisual messaging, as well as e-commerce and digital transactions. For example, users may be able to acquire, show off, and share digital or physical assets associated with the user and/or animal profile via the platform. For example, a dog walker may be able to award a "Good Boy" award to one client animal per week, said award demonstrable on the animal's profile in the form of a badge, accolade, frame, or other visual asset.

In another embodiment, a still further variant on the aforementioned multi-channel data aggregation system and method may be implemented to store and manage animal-related information and animal owner information in association with a digital marketplace platform comprising a plurality of customized user interfaces each configured to link a host server associated with the digital marketplace platform to one or more user devices. A data storage network is functionally linked to the host server and configured to associate primary stored data for an individual non-human animal with respective caregivers, owners and/or breeders, and to further associate derivative stored data with at least familial non-human animal relations and their respective caregivers, owners and/or breeders. The digital marketplace platform is further configured to: generate one or more animal-related digital assets; store said one or more digital assets in association with a corresponding one or more first users; enable designation, by said one or more first users via a respective user interface, of access by other individual users to selected primary and/or derivative data with respect to the one or more animal-related digital assets; and administer transactions between the one or more first users and one or more second users with respect to the one or more animal-related digital assets.

The digital marketplace may generally enable various users to upload animal-related information and selectively share such information with users or user groups; to engage in transactional contracts and smart contracts for ownership and intellectual property; to purchase, transfer, and license digital assets; to transfer monetary value into one or more digital assets; and to manage ownership and co-ownership rights for current and future animals. Data may be shared directly and in full, or data may selectively be shared such that other users may only have access to a subset or obfuscation of data. Further in said example, the user may allow limited access to certain animal information, such as allowing owners of associated animal relatives to know the type and severity of a medical diagnosis but not the diagnosis itself, or to allow unassociated breeders access to anonymized statistical data regarding the animal breed/type and related diagnoses in aggregate.

The digital marketplace platform may facilitate transactional activities for one or more users, including between users. For example, the digital marketplace may permit the sale of animals; the sale of animal-related rights, such as breeding rights; the sale or leasing of digital assets, such as games, badges, etc.; the sale of services such as grooming and training; and the sale of physical assets such as toys, pet care products, and the like. Animal-related digital assets may include intellectual property corresponding to one or more animals. Assets may be related. For example, a physical toy purchased via the platform may also result in the generation of a digital representation of said toy or other digital token. The platform may further programmatically identify related animal and user profiles and update the data accordingly. For example, the production of a foal may automatically verify and populate the data of associated relative animal profiles with the new offspring information; the subsequent performance of one horse within a bloodline may update the associated animal profiles with such information, such as, for specific example, to identify relative likelihoods that related horses will have higher performance based on genetic relatedness. Such information may be protected from disclosure such that one or more users must provide permission before sharing animal or user information. For example, a user may affirmatively post information and choose which users and/or groups with which to share the information, may non-affirmatively share said information based upon the user's privacy settings, or may share said information upon the approval of one or more other users, such as a veterinarian or breeder.

In one aspect, the platform may further enable partial or micro-ownership of an animal or animal-related right. For example, the platform may permit multiple users to purchase or "crowdfund" a particular animal in exchange for a fractional ownership of the animal's breeding rights and/or lineage rights, whereby the users may receive as compensation future revenues from the animal's breeding fees, race performance awards, etc. and, in one embodiment, the animal's descendants' fees/awards/etc. Such ownership information may be stored in accordance with a centralized or decentralized, digitally managed ledger, such that subsequent financial transactions may automatically be processed via smart contracts and divided amongst the plurality of user investors having related ownership rights. Such micro-transactioning may permit, for example, a breeder to fund training for an unproven racehorse while allowing users to diversify their risk via equity crowdfunding investment.

In a further aspect, the present invention relates to a multi-channel data aggregation method according to the following claim 15.

The method, according to the invention, allows communicating non-human animal data among a plurality of remote user networks, wherein at least one user is provided for a particular non-human animal among each of a caregiver user network, an owner user network and a source user network.

The method, according to the invention, comprises:
providing a plurality of user interfaces respectively associated with each of the caregiver user network, the owner user network and the source user network, wherein each user interface is configured to:
link a hosted server network to one or more user devices for the respective user network, and
enable user entry of primary data for individual non-human animals, said primary data stored in accessible association with the hosted server network;
wherein the hosted server network is configured to:
   associate primary stored data for an individual non-human animal with each respective caregiver, owner, and source, and to further associate derivative stored data with at least familial non-human animal relations and their respective caregivers, owners and sources,
   provide unique identifiers for each respective caregiver, owner, and source,
   enable any one or more users to provide selective access to corresponding primary and/or derivative stored data by a specified one or more of the other users, and
   responsive to a user search request from any one or more of the user interfaces, to produce accessible primary and/or derivative data corresponding to parameters in the search request.

Further characteristics and advantages of the present invention will be more apparent with reference to the description given below and to the accompanying figures, provided purely for explanatory and non-limiting purposes, wherein:
- Fig. 1 is a block diagram representing an embodiment of a system in accordance with the present disclosure;
- Fig. 2 is a flowchart representing an embodiment of a method for detecting and predicting animal-related behavior and activity in accordance with the present disclosure;
- Fig. 3 is a flowchart representing an exemplary association of a plurality of animal profiles in accordance with the present disclosure;
- Fig. 4 is a flowchart representing an embodiment of a machine learning method for medical issue risk determination in accordance with the present disclosure;
- Fig. 5 is a flowchart representing an embodiment of a method for aggregating animal information and associated transactions as disclosed herein;
- Fig. 6 is a block diagram representing system-permitted relationship interaction models between users in accordance with the present disclosure;
- Fig. 7 is a block diagram representing an exemplary profile relationship.

The following terms take at least the meanings explicitly associated herein, unless the context dictates otherwise. The meanings identified below do not necessarily limit the terms, but merely provide illustrative examples for the terms. The meaning of "a," "an," and "the" may include plural references, and the meaning of "in" may include "in" and "on." The phrase "in one embodiment," as used herein does not necessarily refer to the same embodiment, although it may. Depending on the embodiment, certain acts, events, or functions of any of the algorithms described herein can be performed in a different sequence, can be added, merged, or left out altogether (e.g., not all described acts or events are necessary for the practice of the algorithm). Moreover, in certain embodiments, acts or events can be performed concurrently, e.g., through multi-threaded processing, interrupt processing, or multiple processors or processor cores or on other parallel architectures, rather than sequentially.

The various illustrative logical blocks, modules, and algorithm steps described in connection with the embodiments disclosed herein can be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing a departure from the scope of the disclosure.

The various illustrative logical blocks and modules described in connection with the embodiments disclosed herein can be implemented or performed by a machine, such as a general purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general purpose processor can be a microprocessor, but in the alternative, the processor can be a controller, microcontroller, or state machine, combinations of the same, or the like. A processor can also be implemented as a combination of computing devices, e.g., a combination of a DSP and a microprocessor, a plurality of microprocessors, one or more microprocessors in conjunction with a DSP core, or any other such configuration.

The steps of a method, process, or algorithm described in connection with the embodiments disclosed herein can be embodied directly in hardware, in a software module executed by a processor, or in a combination of the two. A software module can reside in RAM memory, flash memory, ROM memory, EPROM memory, EEPROM memory, registers, hard disk, a removable disk, a CD-ROM, or any other form of computer-readable medium known in the art. An exemplary computer-readable medium can be coupled to the processor such that the processor can read information from, and write information to, the memory/ storage medium. In the alternative, the medium can be integral to the processor. The processor and the medium can reside in an ASIC. The ASIC can reside in a user terminal. In the alternative, the processor and the medium can reside as discrete components in a user terminal.

Conditional language used herein, such as, among others, "can," "might," "may," "e.g.," and the like, unless specifically stated otherwise, or otherwise understood within the context as used, is generally intended to convey that certain embodiments include, while other embodiments do not include, certain features, elements and/or states. Thus, such conditional language is not generally intended to imply that features, elements and/or states are in any way required for one or more embodiments or that one or more embodiments necessarily include logic for deciding, with or without author input or prompting, whether these features, elements and/or states are included or are to be performed in any particular embodiment.

The term "user interface" as used herein may unless otherwise stated include any input-output module with respect to the hosted server including but not limited to web portals, such as individual web pages or those collectively defining a hosted website, mobile device applications, telephony interfaces such as interactive voice response (IVR), and the like. Such interfaces may in a broader sense include pop-ups or links to third party websites for the purpose of further accessing and/or integrating associated materials, data or program functions via the hosted system and in accordance with methods of the present invention.

The term "communications network" as used herein with respect to data communication between two or more parties or otherwise between communications network interfaces associated with two or more parties may refer to any one of, or a combination of any two or more of, telecommunications networks (whether wired, wireless, cellular or the like), a global network such as the Internet, local networks, network links, Internet Service Providers (ISP's), and intermediate communication interfaces.

The term "user" may refer to any one or more animal care providers, including but not limited to animal owners, animal breeders, veterinarians and veterinary staff, trainers, fosters, custodians, and other animal-associated individuals.

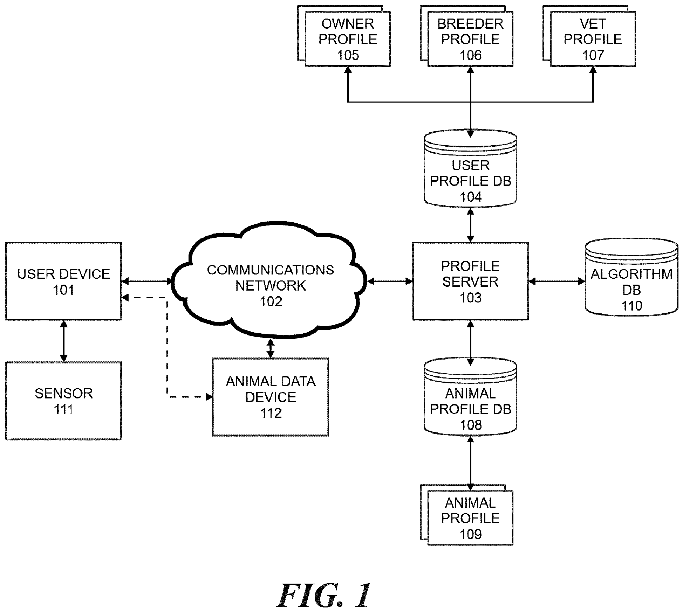

Fig. 1 is a block diagram representing an embodiment of a system in accordance with the present disclosure. A user device 101 associated with a user may be communicatively connected via a communication network 102 to a profile server 103, whereby a user may access data stored in associated with the profile server 103 via the user's user device 101. Data exchange may be provided by and facilitated via a social media platform, whereby a user logs in to the platform provided by the profile server 103 via the user device 101. The profile server 103 may be associated with a user profile database 104 upon which a plurality of user profiles (105, 106, 107) may be stored. In various embodiments, user profiles may be categorized as owner profiles 105, breeder profiles 106, and veterinarian profiles 107. One or more user profiles may be associated with a user, and the user's platform interaction may be specific to the user profile into which the user logs in. User log-in may require one of or both a traditional log-in token such as a password, key, or biometric input and an animal-based log-in token such as an animal identification token or an animal-biometric input such as a nose print, paw print, or iris scan.

Interaction between the various user devices and profile servers may be provided in an embodiment via web-based platforms and associated communication networks, but in alternative embodiments may be provided via, e.g., mobile applications.

The profile server 103 may further be connected to an animal profile database 108 in which a plurality of animal profile 109 are stored. Animal profiles 109 may each be associated with an individual animal, and each animal profile 109 may further be associated with one or more user profiles, including an owner profile 105, a breeder profile 106, and a veterinarian profile 107.

Animal profiles 109 may further be associated with one another in accordance with genealogical information such that the associated animal's sire, dam, litter siblings, and whelps (if applicable) each have their own individual animal profiles 109 associated typologically in accordance with their respective relationship. Accordingly, the profile server 103 may manage a broad network of associated owner profiles 105, breeder profiles 106, veterinarian profiles 107, and animal profiles 109, and profile data for each profile stored in association therewith.

In various embodiments, users may be permitted to add, modify, or delete user profile data or animal profile data associated with the user. For example, a breeder may sell an animal to a user, whereby the breeder may select the associated animal's animal profile 109 and subsequently associate the animal profile 109 with the purchasing owner profile 105. The breeder's breeder profile 106 would remain in association with the animal profile 109. Similarly, the new owner may engage a local veterinarian for care of the new animal, whereby the owner may associate the animal profile 109 with the selected veterinarian's associated veterinarian profile 107.

Various user information may be stored in association with each of the user profiles in accordance with the user profile type. For example, owner profiles 105 may be associated with owner-specific information which may be stored in accordance with the respective user's profile preferences and interactions with the profile server 103. Said owner-specific information may be configured specifically for owner profiles 105 and not, for example, breeder profiles 106 or veterinarian profiles 107, which may be uniquely configured from one another respectively. For example, owner profile information may be stored in a manner facilitating a pet-oriented social media platform, and the owner-user's interaction with the profile server 103 via the owner-user's user device 101 may resemble such software experience accordingly. Similarly, breeders may instead experience a breeder-based user experience providing information specific to and important for breeders, and veterinarians may experience a veterinarian-based user experience providing information specific to and important for veterinary healthcare providers.

In various embodiments, animal profiles 109 may be associated with animal-specific non-medical and medical information provided by associated users' interactions. For example, an animal's breeder may initially upload to the profile server 103 animal profile information such as an animal's breed and lineage; the animal's associated owner may upload additional information such as the animal's name, photos, statuses, and interests; and the animal's associated veterinarian may further upload electronic health record information such as appointments, diagnoses, and immunization for the animal; all of which is stored in association with the animal's respective animal profile 109.

The profile server 103 may be configured to allow users to message, communicate, and/or share profile information with one another. For example, users associated via an animal profile 109 may be permitted to message one another or permit the exchange of information about the associated animal. In various embodiments, visibility of or access to certain profile information may be limited based upon relationship data and/or privacy settings. For example, an owner may explicitly permit or deny certain information of an animal to be shared with the animal's associated breeder and/or veterinarian, or users further associated with those associates (e.g., owners of a specific animal's litter mates).

In an embodiment, the profile server 103 may further be connected to an algorithm database 110 upon which various data aggregation, trend analysis, activity analysis, and behavior analysis algorithms may be stored. The profile server 103 may be configured to execute said algorithms periodically, whereby a plurality of profiles and associated profile data thereof are queried, aggregated, and analyzed for determination of the presence, absence, or statistical likelihood of a particular trend, behavior, or activity. For example, the profile server 103 may, upon request of a breeder, determine all animal profiles for animals related to a breeder's animal, determine all animal profiles for animals related to and including a target breed-mate animal, analyze the associated animal profile data for medical information, and determine a genetic risk report for theoretical offspring of a potential breeding between the breeder's animal and the potential breed-mate animal. Such analysis may, for example, determine a statistical likelihood of one or more desirable or undesirable traits which may be reported to the breeder for consideration. As another example, the system may determine from the user device 101 whether a user is walking an animal.

In some embodiments, the profile server 103 may employ a neutral network engine in association with the algorithm database 110 wherein combinations of aggregate data may be analyzed periodically for trends or correlations. For example, results of algorithmic comparisons may identify that animals that live in a certain region are more likely to exhibit a particular medical symptom than similar animals that do not live within the region, or a particular trait within a breed such as a curly tail or sloped brow may be associated with a particular temperament of the animal. In an embodiment, the neural network engine may be enabled with software and algorithms to determine genetic statistical probabilities based on at least Mendelian characteristic sets and Mendelian inheritance. For example, a breeder with a bitch of one genetic characteristic wanting to achieve a certain genetic characteristic in the bitch's litter may be provided with stud dog recommendations based upon the likelihood of achieving the desired genetic characteristic based upon the combination of the bitch's and stud dog's dominant and recessive traits. In further embodiments, comparisons may be based on non-Mendelian or non-medical profile information extrapolated from user interactions. For example, the system may identify an association between recent diagnoses of a particular intestinal illness with a toy brand determined from natural language processing of owner posts discussing recent purchase of or playing with toys of said brand, thereby generating an alert that there may be a product-liability problem with said toy brand.

In various embodiments, the profile server 103 may be configured to receive telemetric and other sensorial data from one or more sensors 111 associated with the user device 101. For example, the profile server 103 may receive telemetric, barometric, geolocational, and other information from the user device 101 and in accordance with the algorithms of the algorithm database 110 determine that a user is playing fetch with the user's animal. The profile server 103 may, in an embodiment, update the owner profile 105 and/or the animal profile 109 with such determination of activity, such as to record the activity, length, duration, location, and the like. In one embodiment, the profile server may perform and execute the algorithmic determination of activity and/or behavior. In an alternative embodiment, said execution and performance may be performed on the user device 101 and forwarded via the communications network 102 to the profile server 103.

In various embodiments, the profile server 103 and/or user device 101 may further be communicatively connected to one or more animal data devices 112 capable of providing animal-related data to the profile server 103 and/or user device 101. For example, an animal data device 112 may include a GPS tracker, an animal activity tracker, a smart food bowl, a smart water bowl, a smart toy, etc. The animal data device 112 may collect telemetric and other sensory data and forward it to the user device 101 and/or profile server 103 for determination in accordance with the one or more algorithms on the algorithm database 110 for prediction of activity and/or behavior.

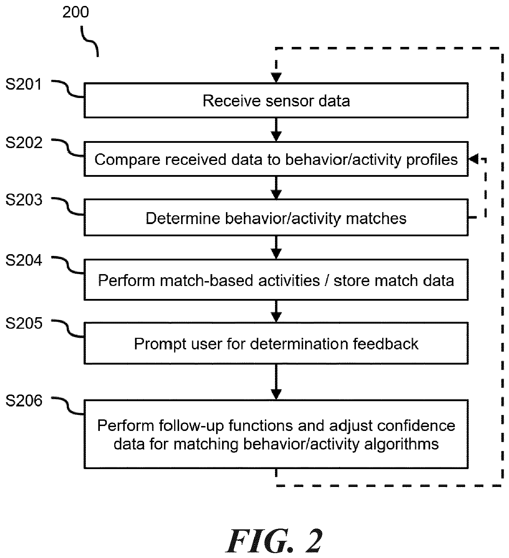

Fig. 2 is a flowchart representing an embodiment of a method for detecting and predicting animal-related behavior and activity in accordance with the present disclosure. The method 200 begins at a first step S201 when the platform receives potential activity and/or behavioral data from any one or more communicatively connected sensors. For example, the system may receive barometric, pedometric, geographic, accelerometric, and other information from a user's smartphone when the user is walking a dog. The data may be uploaded continuously or periodically and may be automatic or at the user's election. In step S202, the system algorithmically compares the received data against one or more activity and behavioral profiles stored upon the system to determine a threshold match. For example, the server may have various data models stored to in association with activities such as walking, resting, sleeping, sitting, playing, working, herding, roaming, etc.; and may have various data models stored in association with behaviors such as excited, happy, sad, angry, scared, threatened, etc. In an embodiment, models may be associated in matrix such that an activity may have one or more associated behavior subtypes, or vice versa. For example, an excited animal walking may generate a different set of sensorial data from that of a bored animal walking based upon speed, acceleration, gyroscopic forces, etc. The data comparison may be made in accordance with one or more algorithmic models for determining activity and/or behavior from one or more user-centric and/or animal-centric sensors. For example, the system may associate a particular data input type with the unique gate of an individual who is walking one dog, a plurality of dogs, etc.

In step S203, the system determines from the comparison whether a given activity is likely to be occurring based upon similarity of the data match. The determination may in an embodiment be made in association with a threshold confidence level. In various embodiments, multiple matches may be considered and made collectively or iteratively. For example, the system may determine highest-confidence matches for "at park," "moving," "happy," "excited;" high-confidence matches for "playing," "running," "jumping," "wagging;" mid-confidence matches for "playing catch" and "playing fetch," and moderate-confidence matches for "playing fetch with stick" and "playing fetch with ball;" with no-confidence or non-match determination for moods/activities such as "sleeping" or "unhappy."

In step S204, the system may perform one or more associated match-based activities. For example, the system may store the determined matches in accordance with either the user profile or animal profile, or both. Various metadata in association with the determined behavior and/or activity may be stored in accordance with the match. For example, time, date, geography, heartrate, activity level, etc. may be determined or provided to the server and stored in association with the user and/or animal profile. In an embodiment, the system may perform other activities such as providing feedback to the user, broadcasting the activity on a social media platform, sending data to a third-party application, modifying a user-experience element on an associated user device (e.g. launching an augmented reality game or checking off a to-do list entry), and the like.

In step S205, the system may provide the user with a prompt for feedback. In an embodiment, the system may query whether the activity and/or behavior detected is correct. For example, the system may prompt the user, "It looks like you are playing fetch with Rex. Do you want to update Rex's play-time profile?" In other embodiments, the system may verify the activity and/or behavior determination indirectly and not specifically through user input, such as via additional sensory input, non-input following notification, etc.

In step S206, the system receives user input or non-input from the query of step S205 and performs one or more follow-up functions in accordance with the received data. For example, the system may update user and/or animal profile information at the confirmation of the user. In various embodiments, the system may adjust the one or more confidence algorithms for determining behavior and activity on the basis of user input. For example, an affirmed identification may result in the adjustment of one or more confidence variable values upwards to improve the degree of confidence or reduce the threshold requirement, whereas a denied identification may result in the reduction of one or more confidence variable values or an increase of the threshold requirement to reduce the likelihood of incorrect matches in future iterations. In an embodiment, the system may adjust the composition of the algorithms themselves as opposed to weighting the variables based upon machine learning. In an embodiment, the system may adjust algorithms for sub-population groups such as for the user-pet, the breed, the animal type, a user-animal type subset, a geography, etc., such that confidence intervals may be different depending upon similarity matches between user and animal data types. For example, the system may have different confidence intervals for young women walking large dogs and men walking cats by comparison to an overall population model of user walking an animal, where the determination of young women walking large dogs is significantly easier and more accurate than for men walking cats.

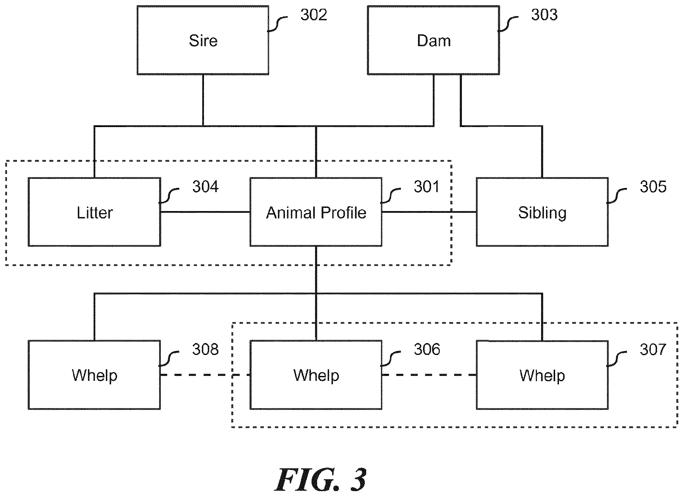

Fig. 3 is a flowchart representing an embodiment of a method for associating a plurality of animal profiles in accordance with the present disclosure. For a given animal profile 301, said animal profile 301 may have an associated sire animal profile 302 and dam animal profile 303, each profile respectively associated with the biological sire and dam of the animal associated with the animal profile 301. In an embodiment, the animal profile 301 may have further associated grandsire and granddam animal profiles, great-grandsire and great-granddam animal profiles, and so forth, such as to establish a pedigree lineage of associated profiles. The animal profile 301 may further be associated with one or more litter animal profiles 304, the one or more litter animal profiles 304 associated with animals born of the same litter as the animal associated with the animal profile 301. Still further, the animal profile 301 may be associated with one or more sibling animal profiles 305 and wherein the sibling animal profiles 305 are associated with animals born of the same sire, same dam, or both, but are not of the same litter as the animal associated with the animal profile 301.

In embodiments where the animal associated with the animal profile 301 has sired or birthed a litter, the animal profile 301 may be associated with one or more whelp animal profiles 306-308, wherein the whelp animal profiles are associated with the offspring of the animal associated with the animal profile 301. The association may be iterative, such that any animal profile itself may be selected as a given animal profile 301. For example, where whelp profile 306 is selected as the iterative profile, the animal profile 301 would instead be the relatively associated sire or dam profile, whelp profile 307 would instead be a relatively associated litter animal profile, and whelp profile 308 would instead be a relatively associated sibling animal profile.

In various embodiments, users may be able to purchase lineage-related rights in part or in full. For example, a user may be able to purchase a partial or full ownership of the animal 301, whereby the user collects one or more of: (1) a percentage of future stud royalties for the animal 301; (2) a percentage of future earnings associated with the animal 301, such as race winnings; (3) a percentage of ownership of the offspring of animal 301 (whelps 306-308); (4) subsequent royalties and/or percentage earnings of the offspring of animal 301; (5) ownership and/or royalties and earnings from subsequent whelps of the whelps 306-308; etc. Such ownership rights may be managed and stored on the system and platform in accordance with one or more animal ownership management algorithms, thereby permitting the system to automatically determine and transact transactions via smart contracts as they occur in the future. For example, said owner of the sire 302 may similarly own an invested portion of said rights for animal 301 and its whelps 306-308, whereby the owner also receives a partial payment for the same transactional activities.

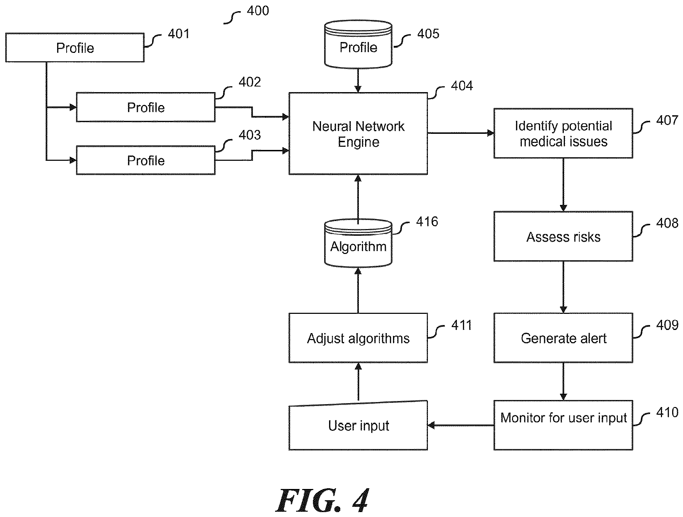

Fig. 4 is a flowchart representing an embodiment of a machine learning method for medical issue risk determination in accordance with the present disclosure. The method 400 begins at a first step S401 when an animal profile is determined for processing. The animal profile may be user-selected or determined in accordance with one or more algorithms stored on the algorithm database. The animal profile may further be a profile stored upon the profile database or alternatively a theoretical profile such as, for example, a profile created in the system's memory as a theoretical whelp animal profile of a given breed pairing.

The system then inputs the breed information (S402) and medical information (S403) of the selected profile into the neural network engine (S404) along with profile trend information determined from the profile database (S405) and in accordance with the algorithm database (S406) and processes said inputs to determine one or more potential medical issues (S407). The system may further determine from the algorithms of the algorithm database a relative probability of each of the medical issues determined (S408). In an embodiment, the probability for each medical issue may be determined from at least the correlation of animal profile information stored in the profile database wherein a high correlation of breed information characteristics with medical issues will be afforded a relatively higher probability.

Upon determination of the potential medical issues and, in some embodiments, the relative probability for said potential medical issues, the system generates alerts based upon the presence and probability of potential medical issues (S409). For example, one or more alerts may be generated upon the user interface for an animal profile with a relatively high risk of one or more of: hip dysplasia, cardiovascular disease, eczema, glaucoma, inguinal hernia, kidney disease, quadriplegia and amblyopia, urolithiasis, and so forth. Alternatively, an alert may not be generated but a breed pairing may be disqualified based upon the one or more determined risks.

In step S410, the system may monitor over the life of the animal associated with the animal profile the medical history inputted by an associated user for determining whether the algorithms predicting associated risks are accurate. The system may further adjust the algorithms stored in the algorithm in accordance with whether the predicted risks become true or not (S411). In an embodiment, variable weights for one or more algorithms may be modified based upon determinations of accurate predictions. In another embodiment, variable weights may not be modified, but correlations may be made stronger or weaker based upon the addition of medical issues for new and existing animal profiles stored in the profile database. For example, an algorithm predicting a high likelihood of arthritis for a particular animal profile may be adjusted as a more accurate predictor where a medical issue of arthritis is subsequently entered in association with the medical history of the animal profile or, alternatively, less accurate if no medical issue of arthritis is subsequently entered, the algorithm's predictive functions and associated variable weights adjusted accordingly.

In an embodiment, said neural network engine and associated algorithms may be used to determine issues other than medical issues such as, for example, breeders associated with disfavored breeding practices. In said embodiment, algorithms may be used to determine breeder rankings and associated alerts based upon correlation of various animal and breeder profile information. For example, breeders who breed and sell an abnormally high volume of animals, as well as breeders who breed animals with abnormally high health issues, may be flagged for alert generation for users.

In a further embodiment, algorithms may be used to identify corollary trends between medical and non-medical issues or non-medical and non-medical issues. For example, said algorithms may be used to determine a correlation between pancreatitis diagnoses and a particular food, or, as another example, between disposition and determined time spent near other animals. Said algorithms may further identify corollary trends affecting animal performance information, such as positive or negative trends between training methodologies and animal race performance.

In various embodiments, method 400 may further be interpreted in instances of behavior and activity detection and may generally be interpreted as an implementation of method 200 described above. In such embodiments, steps S407 and S408 may generally be interpreted as identification of behaviors and activities, not strictly identification of medical issues, and risk assessment may be generally interpreted as assessment of associated activity vectors. However, medical risks and non-medical risks may further be contemplated in association with the determined behaviors and activities. For example, the system may alert a user before an animal becomes dangerously exhausted during a strenuous hike, or that a detected fear and anxiety response in a crowded setting may result in the animal becoming aggressive to other people or animals.

In various embodiments, users may provide and upload information in the form of one or more transactions to be aggregated into blockchain blocks for recordation in a distributed ledger. Transactions may include profile information and may further include transactional information pertaining to profile information, such as the provision of animal-related products or services in association with one or more users and/or user-associated animals. In an embodiment, the system may collect information from third-party sources in association with a user or animal such as electronic medical records, phone applications, pet adoption sites, and the like via one or more APIs. For example, the system may upload animal information in association with a lost/found status for the recordation of animals listed as lost/found and their characteristics. In said example, users who have found animals in their custody may be able to search for lost animals matching similar characteristics and may be able to interact with associated users of said lost animal, such as to send a message of a possible match. Conversely, users who do not match lost-found profile information may be prohibited from accessing or otherwise using associated animal information. In various embodiments, the system may alert one or more users to the absence of certain animal profile information and request the addition of missing information. The system may alternatively, or additionally, query and resolve missing or conflicting information via one or more ledger nodes where such information is present.

The system may further permit user-to-user interactions via the platform. For example, breeders may be permitted to advertise animals for sale to interested potential owners, and veterinarians may be able to advertise veterinary services to owners and/or breeders. Transactions may further be performed and recorded via the platform. Interaction information may be stored and recorded upon the distributed ledger for subsequent access and verification. For example, advertisements and information corresponding thereto, such as timestamps, placement, and content, may be stored within the blockchain for subsequent verification and review. Users may permit or be permitted to message one other in associated with an animal or an animal's familial group. User communications may be cryptographically protected via a public/private key system or other data protection methodologies. For example, an owner may permit photos taken or a medical diagnosis of the owner's animal to be shared with owners, breeders, and/or veterinarians of animals from the same litter. Users may further be able to review and/or provide feedback to other users with respect to services or transactions.

Veterinarians may be able to provide telemedicine to owners via the platform, implementing not only the video capture and streaming capabilities but also data-driven health tracking features, historical records for the animal and/or familial relations, and even historical physical tracking features as may be available using animal sensors or the like. Breeders may search other animals and petition other breeders for potential breeding opportunities.

In various embodiments, when users restrict the sharing of certain content or user/animal profile information with other users, the platform may be configured to prevent visibility of said information by certain users but may otherwise consider said information in determination of trend data and present algorithmic inclusions which factor in such information to a user who may otherwise not have authorization to access the source data. For example, blockchain data may be cryptographically protected such that the encrypted block ledger is generally available but not publicly readable. Users with public/private key access may be able to decrypt block data, either partially or in full depending upon access key rights, to access certain block information. In various embodiments, the ledger may provide for cryptographic information analysis via a threshold cryptosystem, enabling users to access trend data results from the encrypted block information but otherwise preventing users to access personal health or personally identifiable information of other users. In further embodiments, the platform may modify the display of algorithmically determined conclusions when the display of such conclusions could result in the de-anonymization of source data. For example, data queried from limited sample sets (e.g. litter traits) may be displayed as a broad range (e.g. "33-52% likelihood of factor") or a subjective categorization ("moderately high risk") that does not result in reverse-engineering of restricted data.

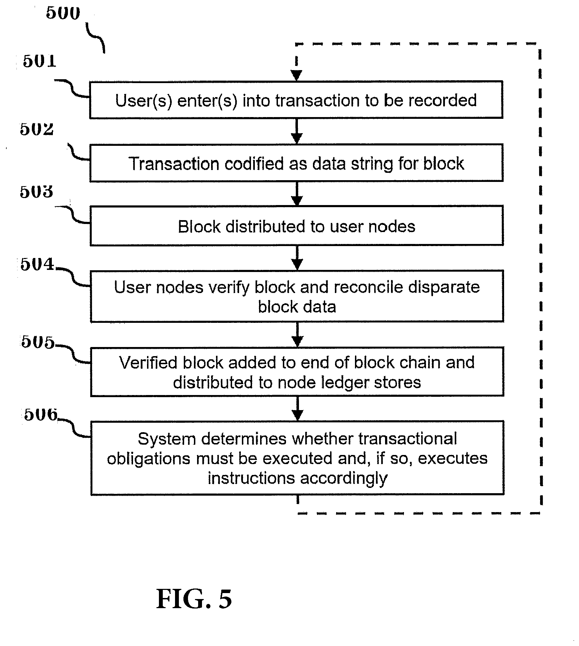

Fig. 5 is a flowchart representing an embodiment of a method for aggregating animal information and associated transactions into a distributed ledger. The method 500 begins at step 501 wherein one or more users enter into a transaction. A transaction may be the addition or modification of animal-related or user-related information, may be a contractual relationship between two or more users, a message, or other such events where user and/or animal information is added or changed. In step 502, one or more transactions are interpreted in accordance with one or more block transaction algorithms and is codified as a unique data string. The data string may be deemed a "block" to be aggregated as part of a blockchain. In step 503, the block representing the one or more transactions is distributed to one or more users in association with data storage for data verification. In step 504, the one or more users to whom the block has been distributed indicate their approval of the transaction data string and verify the block. The verification step may be performed in accordance with one or more verification algorithms, which may include defined mechanisms for determining how to verify or reconcile conflicting or disapproved data. For example, the system may only allow for verification of a block if a plurality of users (i.e. "nodes") agree to approve the transaction.

In step 505, the approved block is added to the blockchain as an end block, and the ledger of blocks as stored upon the various nodes is updated accordingly. In step 506, the system verifies whether the execution of any transactional obligations is required and, if so, performs them. Method 500 may be repeatable such that the steps and blocks are additive, generating a cumulative ledger for each block.

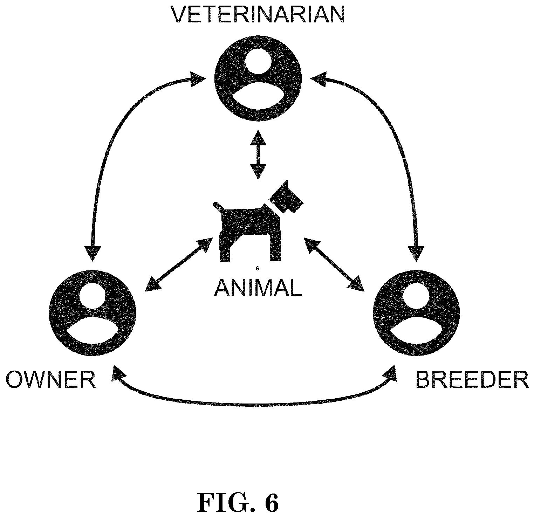

Referring next to Fig. 6, the illustrated block diagram represents system-permitted relationship interaction models between users in accordance with the present disclosure. In an embodiment, users may select a role when signing up for a social media account. The configuration, modules, and data for said user account may be defined in accordance with the type of role selected, and the user may create or otherwise associate with one or more animal accounts. An animal may be associated with one or more users, including owners, breeders, and veterinarians. For example, an owner or breeder may create an animal profile for a new animal upon the system, and a veterinarian may associate with that animal profile where the veterinarian is the primary veterinary care provider for said animal. In various embodiments, other types of owners may be contemplated in the relationship model as discrete entities not pictured but otherwise enabled to interact with other user types. In other embodiments, other types of owners may be categorized as owners, breeders, and/or veterinarians for purposes of interaction models.

Owners may provide owner-oriented information about an animal to the system, including animal interaction information (e.g. walk and play schedules, exercise, diet), social information (e.g. name, temperament, photograph, description), diagnostic information (e.g. mood, illness, injury,) and the like. Breeders may provide breeder-oriented information about the animal, including lineage and genetic information, accolades, pedigree, certifications, etc. Veterinarians may provide veterinarian-oriented information including medical history, health, and the like. Categorical information need not be mutually exclusive. User and system information may be shared and created in real time. Users may further upload this information in the context of a social media log, whereby such information is stored upon a selectively accessible user and/or animal profile.

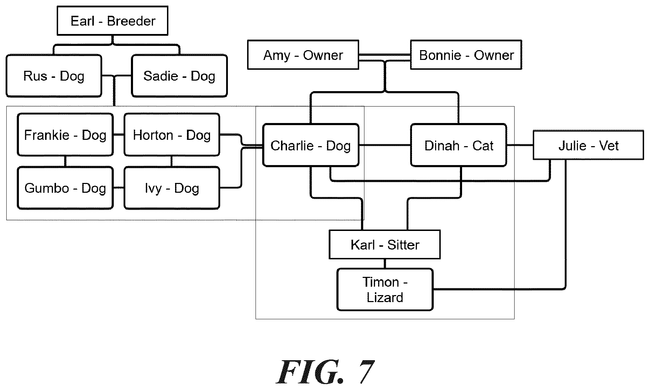

For example, as demonstrated by the exemplary profile relationship of Fig. 7, Amy and Bonnie may be two users, married to one another, who own Charlie the Golden Retriever and Dinah, a domestic longhair cat. In an embodiment, owners Amy and Bonnie may own both Charlie and Dinah jointly, such that each has a 100% ownership right of each animal and any rights thereto. In another embodiment, Amy and Bonnie may own partial percentages for Charlie and Dinah, e.g., each owning 50% of each animal, or, as an alternative example, Amy owning 75% of Charlie and 30% of Dinah, and Bonnie owning 25% of Charlie and 70% of Dinah. In still another example, Amy and Bonnie may own 78.326% of Charlie, whereas the remaining 21.674% of Charlie's ownership is divided amongst other users such as the breeder and micro-owners. As a final example, Amy and Bonnie may own Charlie 100%, whereas one or more other users may own a partial or full interest in Charlie's breeding rights.

In an embodiment, Amy and Bonnie may create user profiles on the transactional platform and associate said profiles as spouses of one another, and/or as co-owners of Charlie and Dinah. Either Amy or Bonnie may create animal profiles for Charlie and Dinah, and the non-creating user may choose to associate her user profile with the animal profile. Charlie may further be associated with Earl, an AKC Golden Retriever Breeder. Pursuant to the examples provided above, Earl may have one or more partial ownership interests of Charlie depending on the transactional sale that occurred for the sale of Charlie. For example, Charlie may have been sold to Bonnie, to Amy and Bonnie jointly, or to a prior owner who then sold or gave away Charlie to Amy and Bonnie. Said transactions may be memorialized on the system in accordance with the transfer of ownership rights contemplated therewith.

Charlie has four associated litter mates (Frankie, Gumbo, Horton, and Ivy) defined as such, and he is further associated with his sibling and cohabitant, Dinah. Amy, Bonnie, Charlie, and Dinah may further be associated with Julie, the family's veterinarian, and Karl, the neighborhood pet-sitter. Amy and Bonnie may be able to transact for Karl's pet-sitting services via the marketplace. For example, Amy and Bonnie may be able to pay Karl on a periodic basis through an application that transfers funds from Amy and Bonnie to Karl periodically and in exchange for pet-sitting services. The application may further provide beneficial transactional and informational services related to the pet-sitting. For example, the application may enable Karl to upload photos of taking Charlie on a walk, or automatically upload playtime activity, or connect into Amy and Bonnie's home alarm system so as to deactivate the home alarm system and unlock the door's smart lock only upon Karl's verified arrival.

In various embodiments, users may provide and upload information in the form of one or more posts to be recorded in association with one or more user or animal accounts. Using the alphabetically oriented example above, Amy may take a photo of Bonnie and Dinah and choose to upload it in association with the profiles of any one or more of Amy's, Bonnie's, Charlie's or Dinah's profiles. Said photo may selectively be shared with any one or more other user and/or animal accounts, such as Julie, Karl, or Lindy, Amy's mom. User profiles may include profile information and may further include transactional information pertaining to profile information, such as the provision of animal-related products or services in association with one or more users and/or user-associated animals. For example, Bonnie and Karl may specify a pet-sitting contract via the platform, and messaging and e-commerce options in relationship to that contract may be provided via the platform to improve the pet-sitting services. As another example, an animal's ownership and rights information may be provided to users such that potential purchasers/investors may see the available ownership rights available for purchase, and under what conditions. In an embodiment, users may be able to see the identity of animal owners and rightsholders, whereas in an alternative embodiment, one or more users may be obfuscated or anonymized so as to protect against the unwanted disclosure of personally identifiable information.

In various embodiments, users may provide and upload information in the form of one or more transactions to be aggregated into blockchain blocks for recordation in a distributed ledger. Transactions may include profile information and may further include transactional information pertaining to profile information, such as the provision of animal-related products or services in association with one or more users and/or user-associated animals. In an embodiment, the system may collect information from third-party sources in association with a user or animal such as electronic medical records, phone applications, pet adoption sites, and the like via one or more APIs. For example, the system may upload animal information in association with a lost/found status for the recordation of animals listed as lost/found and their characteristics. In said example, users who have found animals in their custody may be able to search for lost animals matching similar characteristics and may be able to interact with associated users of said lost animal, such as to send a message of a possible match. Conversely, users who do not match lost-found profile information may be prohibited from accessing or otherwise using associated animal information. In various embodiments, the system may alert one or more users to the absence of certain animal profile information and request the addition of missing information. The system may alternatively, or additionally, query and resolve missing or conflicting information, such as by referencing an animal's medical record or by using artificial intelligence algorithms to predict a certain information trait (e.g. identifying an animal in a picture as a palomino horse).

The previous detailed description has been provided for the purposes of illustration and description. Thus, although there have been described particular embodiments of a new and useful invention, it is not intended that such references be construed as limitations upon the scope of this invention.

## Claims

1. A multi-channel data aggregation system for communicating non-human animal data among a plurality of remote user networks, wherein at least one user is provided for a particular non-human animal among each of a caregiver user network, an owner user network and a source user network, the system being **characterised in that** it comprises:
a plurality of user interfaces respectively associated with each of the caregiver user network, the owner user network and the source user network, wherein each user interface is configured to:
link a hosted server network to one or more user devices for the respective user network, and
enable user entry of primary data for individual non-human animals, said primary data stored in accessible association with the hosted server network;
wherein the hosted server network is configured to;
associate primary stored data for an individual non-human animal with each respective caregiver, owner, and source, and to further associate derivative stored data with at least familial non-human animal relations and their respective caregivers, owners and sources,
provide unique identifiers for each respective caregiver, owner, and source,
enable any one or more users to provide selective access to corresponding primary and/or derivative stored data by a specified one or more of the other users, and
responsive to a user search request from any one or more of the user interfaces, to produce accessible primary and/or derivative data corresponding to parameters in the search request.

2. The system of claim 1, **characterised in that** the hosted server network comprises a decentralized network platform, and is further configured to:
generate a distributed ledger stored across a plurality of nodes;
provide unique identifiers in the distributed ledger for each respective caregiver, owner, and source;
upon confirmation of a transaction between a plurality of users to upload and verify at least sale and brood information via corresponding ledger nodes, wherein at least the brood information comprises primary and/or derivative data associated with animals in the transacted brood;
programmatically identify animal and user profiles associated with the transaction; and update the sale and brood information accordingly.

3. The system of claim 2, **characterised in that** the hosted server network is configured to require verification of one or more digital certificates in association with the transaction, wherein the confirmed transaction comprises a cryptocurrency transaction.

4. The system of claim 1, **characterised in that** the hosted server network is configured, responsive to the user search request from any one or more of the user interfaces, to decrypt selectively accessible primary and/or derivative data via a master key and provide an output report to the requesting user without providing the decrypted data.

5. The system of claim 1, **characterised in that** the hosted server network is configured to:
receive activity data associated with the given non-human animal via the at least one user interface, the primary data comprising data manually entered via the at least one user interface and data automatically generated or derived from one or more sensors positioned in association with the given non-human animal,
detect a behavior or the likelihood of a behavior associated with the non-human animal, and
provide one or more offers to the user via the user interface, based on the detected behavior or likelihood of the behavior,
wherein the one or more offers comprise one or more of: an interactive user experience corresponding to a type of detected behavior or likely behavior; a digital token associated with performance of the detected behavior or likely behavior; and a modified user experience element on the user device.

6. The system of claim 5, **characterised in that** the hosted server network is configured to:
associate detected behavior or likelihood of behavior with one or more animal-related products, and
determine correlations between a type of detected behavior or likelihood of behavior and the one or more animal-related products.

7. The system of claim 1, **characterised in that** it comprises:
a plurality of user interfaces respectively associated with each of a caregiver user network, an owner user network and a source user network, wherein each user interface is configured to link the host server to one or more user devices for the respective user network;
a data storage functionally linked to the hosted server network and configured to associate primary stored data for individual non-human animals with each respective caregiver, owner, and source, and to further associated derivative stored data with at least familial non-human animal relations and their respective caregivers, owners and sources;
wherein the hosted server network is configured to:
aggregate primary and derivative stored data with respect to each individual caregiver, owner and source and across each of one or more metrics specific to their respective user network,
determine a relative importance of the detected behavior or likelihood of behavior associated with the non-human animal for any one or more of the caregivers, owners, and sources, and
deliver one or more messages comprising customized information regarding the detected behavior or likelihood of behavior to one or more of the caregivers, owners, and sources, based at least in part on the determined relative importance for each potential recipient.

8. The system of claim 1, **characterised in that** it further comprises:
a social media platform comprising a plurality of customized user interfaces each configured to link the hosted server network to one or more user devices for a respective user network;
a data storage functionally linked to the host server and configured to associate primary stored data for an individual non-human animal with respective caregivers, owners and/or sources, and to further associate derivative stored data with at least familial non-human animal relations and their respective caregivers, owners and/or sources;
wherein the social media platform is configured to:
enable tagging by users via a respective user interface of selected primary and/or derivative data with respect to an associated animal, wherein the tags designate access by other individual users to the selected primary and/or derivative data, and
responsive to a user request from any one or more of the user interfaces, to produce primary and/or derivative data corresponding to the request and accessible to the requesting user.

9. The system of claim 8, **characterised in that** the tags are configured to designate access by other individual users to one or more of: all of a selected first set of primary and/or derivative data, a subset of a selected second set of primary and/or derivative data, and an obfuscation of a selected third set of primary and/or derivative data.

10. The system of claim 8, **characterised in that** the social media platform is configured to upload animal activity data from a local data source, and to enable an associated user to designate access to the uploaded animal activity data by a selected group of one or more users or a user network.

11. The system of claim 10, **characterised in that** the social media platform is configured to determine current health conditions for a given animal based at least in part on analysis of the uploaded activity data and stored historical activity data and medical history.

12. The system of claim 10, **characterised in that** the social media platform is configured to:
identify current health conditions for a given non-human animal as being independent to the given non-human animal, common to a breed associated with the given non-human animal, or potentially collective to familial relations of the given non-human animal, and
for identified current health conditions as potential collective to familial relations, to generate notifications comprising accessible subsets or obfuscations of related primary and/or derivative data to user interfaces associated with familial relations of the non-human animal.

13. The system of claim 8, **characterised in that** one or more owner user interfaces are configured to display and transmit image data captured via an image capturing device of an associated owner user device, wherein the social media platform is selectively configured in association with owner user authorization to enable access by other users to the image data.

14. The system of claim 1, **characterised in that** the hosted server network is configured to:
generate one or more animal-related digital assets and storing said one or more digital assets in association with a corresponding one or more first users,
enable designation, by said one or more first users via a respective user interface, of access by other individual users to selected primary and/or derivative data with respect to the one or more animal-related digital assets, and
administer transactions between the one or more first users and one or more second users with respect to the one or more animal-related digital assets.

15. A multi-channel data aggregation method for communicating non-human animal data among a plurality of remote user networks, wherein at least one user is provided for a particular non-human animal among each of a caregiver user network, an owner user network and a source user network, the method being **characterised in that** it comprises:
providing a plurality of user interfaces respectively associated with each of the caregiver user network, the owner user network and the source user network,
wherein each user interface is configured to:
link a hosted server network to one or more user devices for the respective user network, and
enable user entry of primary data for individual non-human animals, said primary data stored in accessible association with the hosted server network;
wherein the hosted server network is configured to:
associate primary stored data for an individual non-human animal with each respective caregiver, owner, and source, and to further associate derivative stored data with at least familial non-human animal relations and their respective caregivers, owners and sources,
provide unique identifiers for each respective caregiver, owner, and source,
enable any one or more users to provide selective access to corresponding primary and/or derivative stored data by a specified one or more of the other users, and
responsive to a user search request from any one or more of the user interfaces, to produce accessible primary and/or derivative data corresponding to parameters in the search request.
